# EUROPEAN PATENT APPLICATION

(11) **EP 4 806 451 A1**
(43) Date of publication of application: **16.09.2026**
(21) Application number: 26163598.1
(22) Date of filing: 10.03.2026
(51) Int. Cl.: A61K 38/00, C12N 9/16, C12N 9/22, A61K 38/46

(54) **BLOCKING AGENT THAT SUPPRESSES VIRUS PROPAGATION, SYNTHETIC NUCLEIC ACID ENCODING BLOCKING AGENT, AND METHOD FOR TREATING AND/OR MITIGATING VIRUS INFECTION BY USING BLOCKING AGENT**

(30) Priority: 11.03.2025 US 202563769796 P
(71) Applicant: National Taiwan University, Taipei City 106319 (TW)
(72) Inventor: Chang, Zee-Fen, 106319 Taipei City (TW)
(74) Representative: Scholz, Volker

(57) **Abstract**

The present disclosure provides a blocking agent that suppresses the propagation of viruses, a synthetic nucleic acid encoding the blocking agent, and the blocking agent for use in the treatment and/or mitigation of virus infection. The present disclosure achieves the effect of treating and/or mitigating virus infection through various experiments.

## Description

### BACKGROUND OF THE INVENTION

### 1. Field of the Invention

The present invention relates to the novel design of viral nucleic acid degraders on mitochondrial surface and their method of use. In particular, it relates to the compositions of the viral RNA degraders and therapeutics that prevent viral RNA replication in host cells. Such novel viral RNA degrader compositions and therapeutics may block viral propagation, which are useful in methods for treating or mitigating viral infection. Therefore, the present invention relates to a blocking agent that suppresses the propagation of viruses and the blocking agent for use in the treatment and/or mitigation of virus infection.

### 2. The Prior Art

RNA viruses have been known to cause a variety of diseases in human and livestock. The lack of proofreading capacity of virus-encoded RNA-dependent RNA polymerase (RdRp) leads to the high mutation rate of viral RNA (vRNA) genome replication. Although anti-viral drugs target viral RdRp to suppress vRNA replication and the vaccines are developed to counteract the infection, the high frequency of viral mutations might still generate the therapy issues during emergent outbreak of infection. Moreover, many RNA viruses still lack vaccine and drug for managing infection.

A number of studies have been performed to screen the host factors hijacked by virus for viral propagation. Targeting these host factors might be developed for anti-virus treatments. However, other host factors that restrain viral replication are often missing in the screening. Increasing expression of these factors could also elicit anti-viral effect.

The infectious cycle of many RNA viruses takes place in the cytosol of host cells. It has been shown that mitochondria play vital role in response to virus infection. Viral RNA released in the host cytosol affects mitochondrial dynamics and metabolism to facilitate their replication. Meanwhile, mitochondria also serve as signaling hubs in immune responses to counteract viral invasion. Whether virus-induced mitochondrial changes might release mitochondrial factor that directly affects viral RNA replication remains uncertain.

The present invention employed vesicular stomatitis virus (VSV) as a study model. VSV belongs to the Rhabdoviridae family, which is a negative-sense single-stranded RNA virus. VSV has a high transmission rate across a wide range of animals. The present invention found that VSV RNA transcription in host cells occurs in the sphere structure consisting of mitochondria. We assumed that mitochondria participate in viral RNA production by providing metabolites including nucleotides and amino acids.

Intriguingly, upon VSV infection, the induction of mitochondrial sphere formation was accompanied by a leakage of endonuclease G (ENDOG) from mitochondria into the cytoplasm. ENDOG, a mitochondrial nuclease, has been shown critical in mtDNA replication and the maturation production of mt-tRNA in mitochondria.

The present invention found that ENDOG knockdown indeed increased viral gene expression, viral replication, and viral titer, suggesting ENDOG is a host factor against viral propagation.

In the meanwhile, we found that ENDOG can cause nuclear DNA damage in host cells after virus infection. Expression of ENDOG deleted of its mitochondrial targeting sequence (MTS) also sustains nuclear DNA damage, suggesting that the bad side of ENDOG to cells outside mitochondria.

An ENDOG is engineered to be able anchored and expressed on mitochondrial surface by replacing its mitochondrial targeting sequence with a mitochondrial outer membrane targeting sequence, thus preventing ENDOG translocated to nuclei and nuclear DNA damage. This design allows ENDOG constantly accessible to viral RNAs to cause their degradation.

Those skilled in the art urgently need to develop a novel medicament for treating and/or mitigating virus infection for the benefit of a large group of people in need thereof.

### SUMMARY OF THE INVENTION

A primary objective of the present invention is to provide a blocking agent that suppresses virus propagation for treating and/or mitigating virus infection, comprising a polypeptide and an enzyme conjugated to the polypeptide, wherein the blocking agent is expressed on a surface of an organelle.

Another objective of the present invention is to provide the abovementioned blocking agent for use in the treatment and/or mitigation of virus infection.

Another objective of the present invention is to provide a synthetic nucleic acid, encoding the abovementioned blocking agent, wherein the synthetic nucleic acid is in the form of a DNA or an RNA.

According to an embodiment of the present invention, the polypeptide is conjugated to the enzyme, anchoring the enzyme to the surface of the organelle, thereby reducing nucleic acid synthesis of the viruses at the neighborhood site of the organelle in host cells.

According to an embodiment of the present invention, the enzyme is anchored to an outer membrane of the organelle.

According to an embodiment of the present invention, the blocking agent inhibits viral production without damaging nuclear DNA (nDNA).

According to an embodiment of the present invention, the virus is an RNA virus or a DNA virus.

According to an embodiment of the present invention, the RNA virus is selected from the group consisting of: dengue virus, Zika virus, vesicular stomatitis virus (VSV), Influenza virus, respiratory syncytial virus (RSV), and a combination thereof.

According to an embodiment of the present invention, the organelle is a mitochondrion, endoplasmic reticulum, or Golgi apparatus.

According to an embodiment of the present invention, the polypeptide is an N-terminal sequence of translocase of outer mitochondrial membrane 20 (TOM20).

According to an embodiment of the present invention, the polypeptide comprises an amino acid sequence of SEQ ID NO: 1.

According to an embodiment of the present invention, the enzyme is endonuclease G (ENDOG).

According to an embodiment of the present invention, the surface of the organelle is an outer membrane of mitochondria.

According to an embodiment of the present invention, the blocking agent is in a dosage form for oral administration.

According to an embodiment of the present invention, the blocking agent is in a dosage form for parenteral administration.

The present invention is also based on the discovery that RNA viruses synthesize their genes or RNAs on mitochondria.

The present invention is based on the discovery that after viral infection, the mitochondria of host cells release a nuclease that breaks down viral RNA, but also causes damage to the nuclear genes of host cells.

Therefore, the enzyme released from the mitochondria is genetically modified by removing its mitochondrial delivery sequence and replacing it with a sequence that can fix the enzyme to the outer membrane of the mitochondria. Since viral genes synthesize new RNAs on the outer membrane of the mitochondria, nucleases fixed on the outer membrane of the mitochondria can cause viral RNA degradation. Therefore, viral RNA replication and transcription are blocked.

The present invention is based on the discovery that viral RNA transcription at mitochondrial spheres accompanied by endonuclease G (ENDOG) leakage from mitochondria that restricts viral propagation. The inventor designs ENDOG expressed on mitochondrial surface and methods of use in treating or suppressing viral propagation.

The present invention provides a composition.

In some embodiments of the present invention, the composition comprises the trans-membrane sequence of mitochondrial outer membrane protein.

In some embodiments of the present invention, the composition comprises endonucleases
In some embodiments of the present invention, the compositions do not lead to cellular RNA or DNA degradation.

In some embodiments of the present invention, the delivery of the engineered composition can be in the forms of DNA construct or RNA, which inside the cell can be translated to the desired engineered enzymes.

The present invention further provides a pharmaceutical composition comprising the compositions described herein and a pharmaceutically acceptable carrier.

In some embodiment, the composition is administered via inhalation, nasal spray, aerosol spray, orally, intravenously, intraperitoneally, subcutaneously, via a sustained-release delivery system or combinations thereof.

In some embodiments, the subject is a mammal.

These and other features, aspects, and the anti-virus effect of the present invention will become better understood with reference of the following description, examples, claims and appended claims.

In summary, the present invention achieves the effect of treating RNA virus infection through the results illustrated in the following examples. In particular, by linking to polypeptide sequences, proteins that can break down nucleic acids or inhibit nucleic acid synthesis can be engineered to anchor on organelle surfaces. Therefore, engineered enzymes directly and negatively impact the nucleic acid synthesis of invaded viruses at the neighborhood site of the organelle in host cells.

This method differs from traditional antiviral small molecule drugs in that it can suppress the propagation of different types of viruses, and is not affected by viral gene mutations or evolution. This organelle-associated vRNA degrader is a drug that combats a variety of viruses and can overcome drug-resistant RNA viruses.

The embodiments of the present invention would be further described below. The following examples are used to illustrate the present invention and are not intended to limit the scope of the present invention. Anyone skilled in the art can make some changes and modifications without departing from the spirit and scope of the present invention. Therefore, the scope of the present invention shall be defined by the appended claims.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.

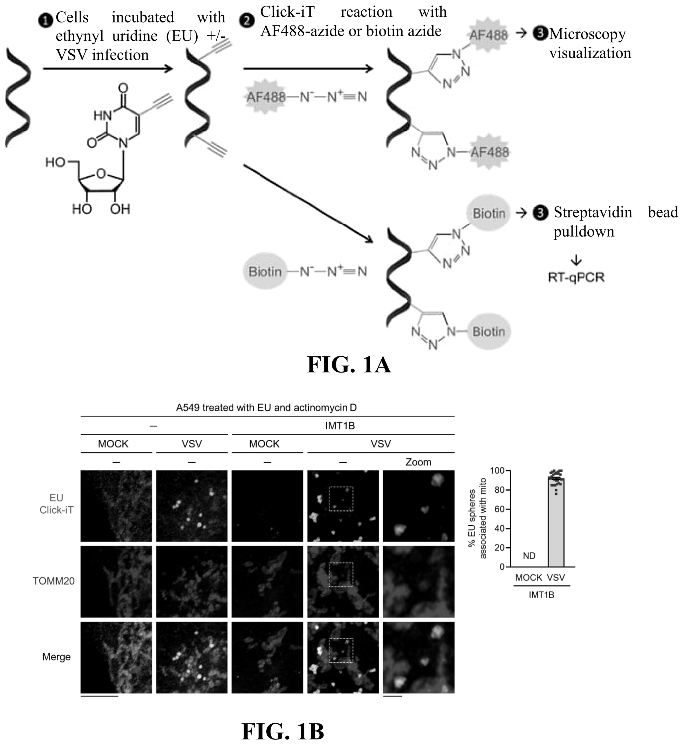

FIGs. 1A-1C show viral RNA synthesis at the induced mitochondrial spheres in host cells. (1A) The workflow for newly synthesis of 5-ethylnyl uridine (EU)-labeled RNA by Click-iT reaction using Alexa488-azide for fluorescence imaging and Biotin-azide for streptavidin-coated beads followed by pulldown and RT-qPCR analysis. (1B) A549 cells were incubated with EU and treated with Actinomycin D (ActD) for mock- and VSV infection in the presence of IMT1B, an inhibitor of mtRNA polymerase, as indicated. At 8h, cells were fixed for Click-iT reaction and immunofluorescence (IF) staining. Representative confocal images of nascent EU-labeled RNA by Alexa-488-azide Click-iT and translocase of outer mitochondrial membrane 20 (TOM20) immunofluorescence are shown (scale bar: 5 µm). (1C) The biotinylated EU-labeled RNAs pulldown by streptavidin beads were analyzed by RT-qPCR for the quantification of *VSV-G, VSV-M,* mitochondrial tRNA *(MT-LT1),* mitochondrial RNA (*MT-ND6*) and β-actin (*ACTB*) RNA levels from three independent experiments (mean ±standard error (SEM), * P < 0.05, ** P < 0.01; ND: not detected; NS: not significant).

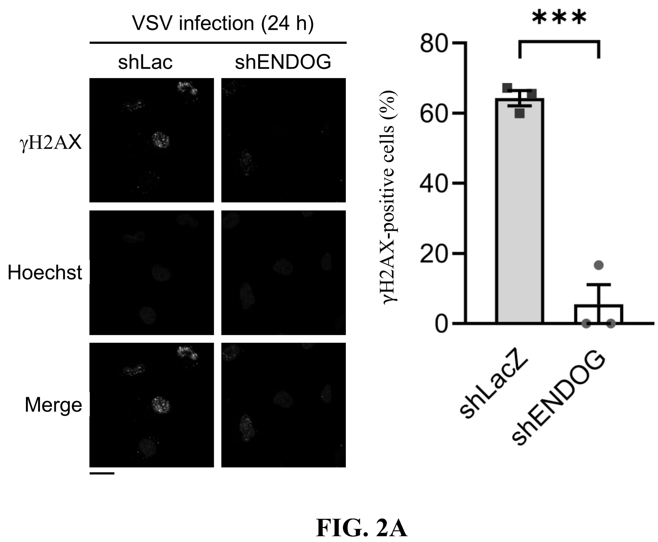

FIGs. 2A-2H show that ENDOG released from mitochondria can act as a host factor that reduces viral RNA propagation but increases nuclear DNA damage. (2A-2B) ENDOG is involved in VSV-induced nuclear DNA damage. (2A) Control and ENDOG knockdown cells were infected by VSV (MOI=0.2). At 24h post infection, cells were fixed for IF staining. Representative confocal IF images of γH2AX IF staining in control- and ENDOG-knockdown A549 cells were shown (scale bar: 10 µm). The bar graph shows quantification of the percentage of γH2AX positive cells by ImageJ from three independent experiments (mean + SEM, ** P < 0.01). (2B) ENDOG knockdown cells were transfected with the plasmid of ENDOG deleted of mitochondrial targeting sequence (ΔMTS-ENDOG). These cells were infected with VSV (MOI=0.2). At 16h post infection, cells were fixed for γH2AX IF staining. Representative images and quantification of percentage of γH2AX positive cells are shown (n=3). (2C-2D) The release of ENDOG from mitochondria after VSV infection. Cells were infected by VSV (MOI=1) for 1h. At 8h post infection, cells were fixed for ENDOG and TOM20 IF staining. (2C) Representative confocal IF images of ENDOG and TOM20 staining in mock- and VSV-infected A549 cells are shown (scale bar: 10 µm). The bar graph shows the quantification of ENDOG intensity outside mitochondria by ImageJ from three independent experiments (mean ± SEM; *** P<0.005). (2D) Western blot analysis of VSV-N and ENDOG in mitochondrial fraction and cytosolic fraction with 0.3% of whole-cell lysates corresponding to the loading amount of mitochondria fraction. TOM20 and TUBB were loading controls for the fractionation. (2E-2G) ENDOG is a host fact that restricts VSV propagation. Control LacZ and ENDOG knockdown A549 cells were infected by VSV (MOI=1) for 1h. At 8h, cells were harvested for (2E) RT-qPCR and (2F) the medium for plaque assay. Fold changes in viral gene (*VSV-G*) levels in mock- or VSV-infected A549 cells and PFU/ml from three independent experiments are shown (mean + SEM, * P < 0.05; NS: not significant). (2G) Cells at the indicated time were fixed for IF staining using dsRNA and COX4 antibody. Left shows the representative IF images. Right shows the quantified data of the dsRNA signal intensity by ImageJ from three independent experiments. (mean ± SEM, * P < 0.05; NS: not significant). (2H) A summary of two different effects of ENDOG released from mitochondria on host cells after virus infection.

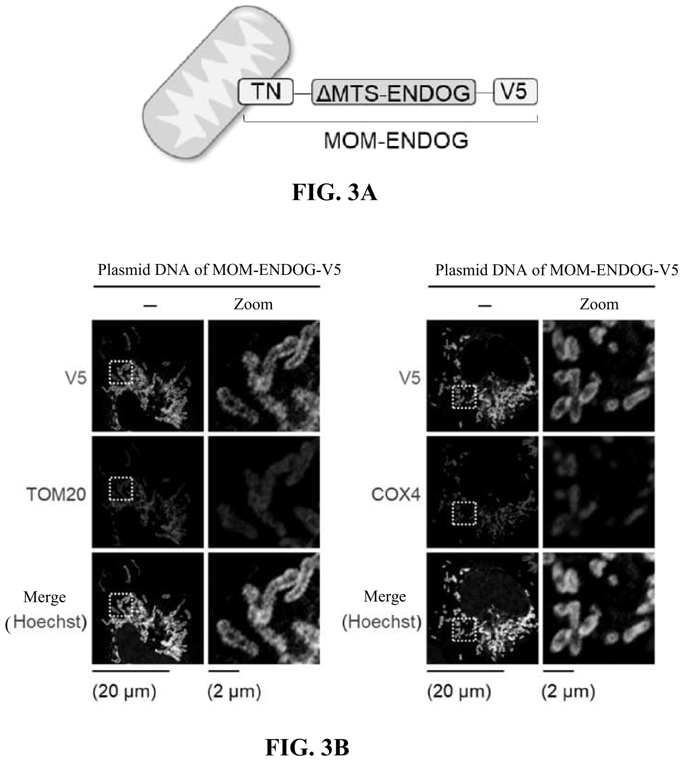

FIGs. 3A-3C show engineering an ENDOG, mitochondrial outer membrane (MOM)-ENDOG, expressed on mitochondrial surface. (3A) The design of ENDOG expressed on mitochondrial surface. The expression of vector of MOM-ENDOG was constructed by replacing the mitochondrial targeting sequence (MTS) of ENDOG-V5 by the N-terminal sequence of TOM20 (TN: SEQ ID NO:1), thus allowing ENDOG expressed on mitochondrial outer membrane (MOM). (3B) Representative super-resolution IF images of A549 cells transfected with TN-ENDOG^{ΔMTS}-V5 (MOM-ENDOG) by V5/TOM20 and V5/COX4 are shown (scale bar: 20 µm). (3C). Mitochondria fractions isolated from MOM-ENDOG-overexpressing A549 cells were incubated with proteinase K as indicated for 30 min in the presence and absence of 0.1% TritonX-100 for Western blot analysis using antibodies of the mitochondrial outer-membrane protein TOM20, matrix proteins and pyruvate dehydrogenases (PDHα/β).

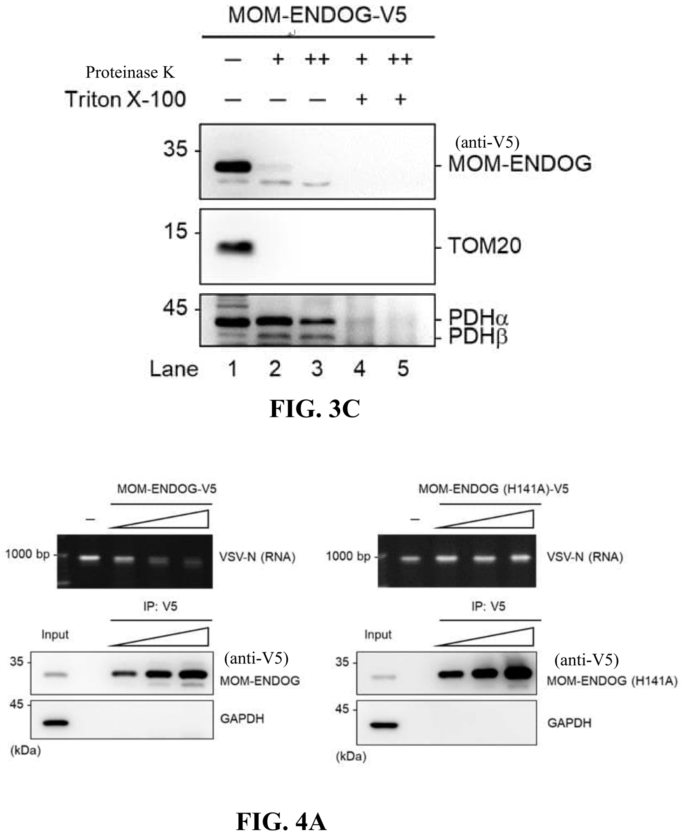

FIGs. 4A-4B show that MOM-ENDOG degrades viral N-RNA and prevents viral N protein expression in host cells. (4A) In vitro degradation of VSV-N RNA by MOM-ENDOG. Cells were transfected with increasing amounts of WT and H141A mutant of MOM-ENDOG-V5 followed by V5 immunoprecipitation. The immunoprecipitates were incubated with VSV N-RNA transcripts obtained from the in vitro transcription by SP6 RNA polymerase of a SP6-plasmid vector with VSV-N gene (1000bp length) insertion. After incubation for 30 min, the reaction products were analysis by gel electrophoresis followed by ethidium bromide staining (left). The immunoprecipitates were analyzed by Western blot (right). (4B) Cells were transfected with the plasmids of MOM-ENDOG-V5 (wild-type vs H141A mutant) overnight and infected with VSV for 1h. Cells were fixed at 8h for IF staining of VSV-N. Representative images (left) and the quantitation of VSV-N IF intensity in V5-positive cells are shown (right). Data are from three experiments (mean ± SEM, ** P < 0.01, ***P< 0.005).

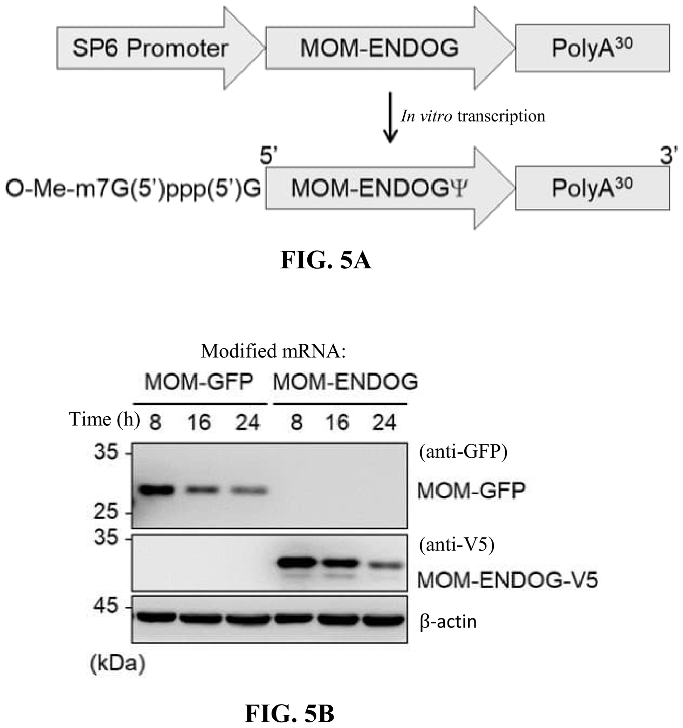

FIGs. 5A-5E show that delivery of the modified mRNA of MOM-ENDOG containing polyA³⁰ tail suppresses VSV propagation without causing nuclear DNA damage. (5A) The generation of MOM-ENDOG modified mRNA. A schematic illustration for generation of MOM-ENDOG modified mRNA containing 5' capping, polyA³⁰ and pseudoUridine (ψ). (5B) A549 cells transfected with 400 ng of modified mRNAs of MOM-GFP or MOM-ENDOG were harvested at indicated time for Western blot. (5C-5E) Cells were infected with VSV (MOI=1) for 1 h and then transfected with modified mRNA of MOM-GFP, WT- and H141A- MOM-ENDOG. (5C) The culture media were collected at 8 h post infection for plaque assay. Plaque forming units (PFUs) of media were determined. Data from three independent experiments are presented (mean ± SEM, * P < 0.05). The expressed levels of MOM-GFP, WT- and H141A-ENDOG were shown by Western blots. (5D) Cells were fixed for dsRNA IF staining. Representative confocal IF images of dsRNA and V5 IF staining at 20 h after mock- or VSV-infection are shown (scale bar: 10 µm). The bar graph shows relative dsRNA signal intensity in transfected cells quantified by ImageJ from three independent experiments (mean ± SEM, ** P < 0.01). (5E) LacZ control and ENDOG-knockdown A549 cells were transfected wit modified mRNAs of MOM-GFP or MOM-ENDOG and infected by virus. At 24h post infection, cells were fixed for γH2AX IF staining. Representative confocal IF images are shown (scale bar: 10 µm). The bar graph shows quantification of the percentage of γH2AX positive cells by ImageJ from three independent experiments (mean ± SEM, ** P < 0.01).

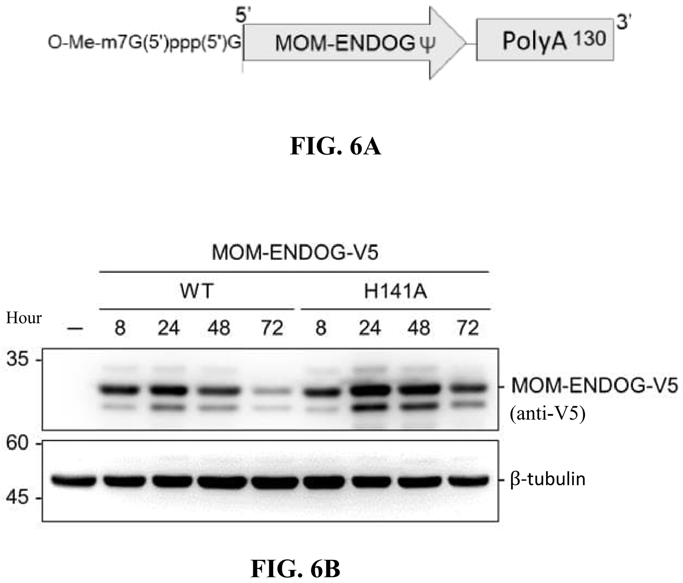

FIGs. 6A-6E show that prolonged duration of MOM-ENDOG markedly reduces VSV virus production. (6A) The design of capping modified mRNA of MOM-ENDOG containing polyA¹³⁰ tail. (6B) A549 cells transfected with 400 ng of capping modified mRNA of WT and H141A-MOM-ENDOG containing polyA¹³⁰ tail were harvested at the indicated times for Western blot analysis. (6C-6D). Cells transfected with modified mRNA overnight were infected by VSV (MOI=0.2) for 1h. Cells and medium were harvested at 24h post-infection for (6C) rt-qPCR of viral genes (VSV-G and VSV-M) and (6D) plaque assay. E. The levels of mt-tRNA, mt-ND4, mtND6, 18S RNA by rt-qPCR in cells transfected with MOM-ENDOG-polyA¹³⁰ for 24h. Data are presented in relative amount normalized by β-actin from three independent experiments (NS, not significant).

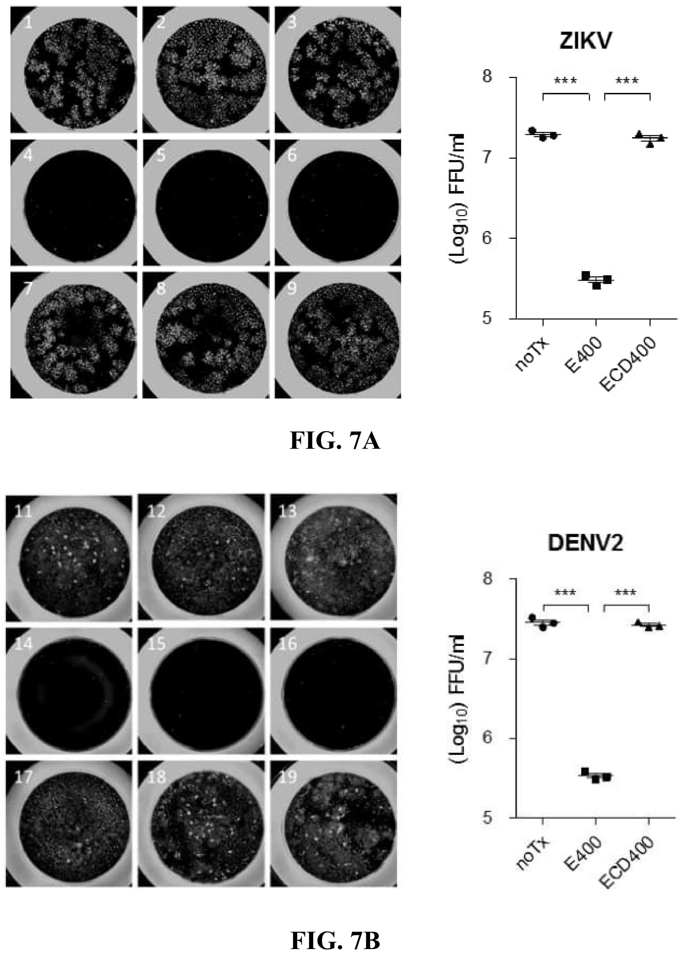

FIGs. 7A-7D show that delivery of the modified mRNA of MOM-ENDOG containing polyA¹³⁰ tail markedly suppresses ZiKa, Dengue and Influenza virus propagation. (7A) A549 cells transfected with 400 ng of capping modified mRNA of WT and H141A-MOM-ENDOG containing polyA¹³⁰ tail. Cells were then infected by Zika virus (ZIKV, MOI=0.1) for 1h. Cells and medium were harvested at 24h post-infection for plaque assay. (7B) A549 cells transfected with the modified mRNA as described above, and infected by Dengue virus 2 (DENV2, MOI=0.1) for 1h. Cells and medium were harvested at 24h post-infection for plaque assay. Data are from three experiments (mean ± SEM, ***P< 0.005). (7C) A549 cells transfected overnight with the modified mRNA, and infected by Influenza A virus (H1N1, MOI=0.5) for 1h. Medium was harvested at 48h post-infection for plaque assay. All quantitative results are expressed as mean + standard error (SEM). NS: not significant, *P< 0.05, **P< 0.01, ***P< 0.001; Two-tailed t-tests were used for comparisons between two groups, and one-way ANOVA was used for comparisons among multiple groups. (7D) The proposed mechanism for MOM-ENDOG-mediated anti-virus activity. After cellular entry, the disassembly of RNA virus releases vRNA and induces mitochondrial spheres as vRNA production factories. MOM-ENDOG expressed on mitochondrial surface by the delivered modified mRNA degrades accessible vRNAs, thus terminating the propagation of vRNA replication.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

### Definition

As used herein, the data provided represent experimental values that can vary within a range of +20%, preferably within +10%, and most preferably within ±5%.

Unless otherwise stated in the context, "a", "the" and similar terms used in the specification (especially in the following claims) should be understood as including singular and plural forms.

Unless otherwise defined, all technical terms used herein have the same meaning as commonly understood by one of ordinary skill in the art to which this invention belongs.

To facilitate understanding of the present application and for ease of reference, a number of terms and abbreviations as used herein are defined below.

As used herein, the term "transfection" and "delivery" are used to describe the process by which a composition of the present invention is transfected or delivered to a subject of performance or a procedure of modality on a subject for the purpose of obtaining a therapeutic benefit of health-related condition. The terms of "transfection" or "delivery" are interchangeable.

As used herein, the terms "suppressing", "mitigating", "reducing", or any variation of these terms, includes any measurable decrease to achieve a desired result.

As used herein, the term "broad-spectrum anti-viral agent" refers to the use of the compositions of the present invention to treat or prevent a subject or a target cell infected by different types of viruses.

As used herein, the terms "therapeutically effective, "therapeutically beneficial", or "prevention" refer to anything that enhances the well-being of the treated subject.

The amount of a composition administered will, of course, be dependent on the subject being treated and the route of administration utilized. The exact formulation, route of administration and dosage can be chosen in view of the treated subject's condition.

While the invention has been described in terms of what is presently considered to be the most practical and preferred embodiments, it is to be understood that the invention needs not be limited the disclosed embodiments. On the contrary, it is intended to cover various modifications and similar arrangements included within the spirit and scope of the appended claims, which are to be accorded with the broadest interpretation so as to encompass all such modifications and similar structures.

The present invention is further illustrated by the following specific examples. The examples are provided for illustration only and should not be construed to limit the scope of the invention in any way.

The present invention is further illustrated by the following examples. These examples are provided for illustration only and are not intended to limit the scope of the present invention. The scope of the present invention is shown in the appended claims.

### Example 1

### Viral RNA synthesis at VSV-induced of mitochondrial spheres in host cells

FIGs. 1A-1C show viral RNA synthesis at the induced mitochondrial spheres in host cells. (1A) The workflow for newly synthesis of 5-ethylnyl uridine (EU)-labeled RNA by Click-iT reaction using Alexa488-azide for fluorescence imaging and Biotin-azide for streptavidin-coated beads followed by pulldown and RT-qPCR analysis. (1B) A549 cells were incubated with EU and treated with Actinomycin D (ActD) for mock- and VSV infection in the presence of IMT1B, an inhibitor of mtRNA polymerase, as indicated. At 8h, cells were fixed for Click-iT reaction and immunofluorescence (IF) staining. Representative confocal images of nascent EU-labeled RNA by Alexa-488-azide Click-iT and translocase of outer mitochondrial membrane 20 (TOM20) immunofluorescence are shown (scale bar: 5 µm). (1C) The biotinylated EU-labeled RNAs pulldown by streptavidin beads were analyzed by RT-qPCR for the quantification of *VSV-G, VSV-M,* mitochondrial tRNA *(MT-LT1*)*,* mitochondrial RNA (*MT-ND6*) and β-actin (*ACTB*) RNA levels from three independent experiments (mean +standard error (SEM), * P < 0.05, ** P < 0.01; ND: not detected; NS: not significant).

The subcellular location of initial transcription of released (-)ss-RNA from the infected VSV particle is unclear. To determine the subcellular location where early viral RNA (vRNA) transcription takes place in host cells, we incubated A549 cells, an adenocarcinomic human alveolar basal epithelial cell line, with ethylyne-uridine (EU) in the presence of actinomycin D (ActD) to block the RNA polymerases in nuclei of host cells after VSV infection. The newly synthesized RNA was measured by EU-mediated-Click-iT reaction for fluorescence imaging and pulldown analysis via streptavidin beads for analyzing the labeled viral RNA by RT-PCR as illustrated (FIG. 1A).

After Alexa-488-azide Click-it reaction and immunofluorescence (IF) staining of TOM20, a mitochondrial outer membrane protein (MOM), the images acquired by super-resolution microscopy revealed EU-labeled RNA signals in filament and spherical shapes highly co-localized with mitochondria in VSV-infected cells. As a contrast, in uninfected cells, only EU-labeled filaments were observed (FIG. 1B). By co-incubation with IMTIB, an inhibitor of mitochondrial RNA polymerase, the EU-labeled RNA signals in filament shape colocalized with mitochondria were diminished in uninfected and infected cells. Of note, IMTIB treatment did not affect the EU-labeled RNA/mitochondria converged in spherical structures in VSV-infected cells, suggesting these signals from virus not from mitochondria.

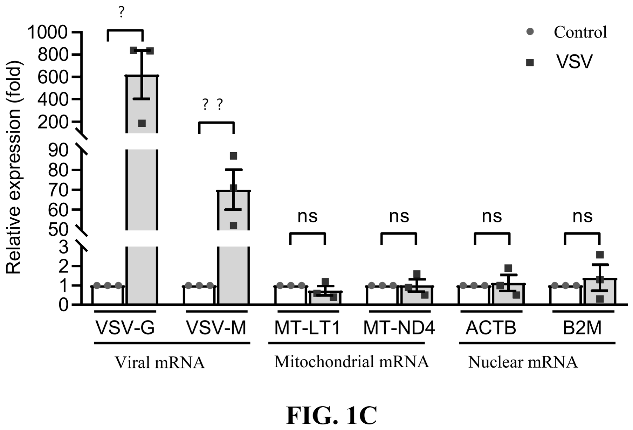

To ensure these EU-labeled spheres containing viral RNA, EU-labeled RNAs by biotin-azide Click-iT reaction were pulldown by streptavidin beads, which were analyzed by RT-qPCR analysis. The results showed high amounts of EU incorporated into VSV G- and M- RNA transcripts, but little into mt-tRNA, mtND4, β-actin, or β2 microglobulin RNA transcripts (FIG. 1C). Because of the inclusion of IMTIB during EU incubation, the streptavidin beads did not pulldown mitochondrial RNA transcripts. These data conclude that upon virus infection, the induced mitochondria spheres are the sites where viral RNAs are synthesized.

### Example 2

### ENDOG released from mitochondria as a host factor that reduces viral RNA propagation but increases nuclear DNA damage

FIGs. 2A-2H show that ENDOG released from mitochondria can act as a host factor that reduces viral RNA propagation but increases nuclear DNA damage. (2A-2B) ENDOG is involved in VSV-induced nuclear DNA damage. (2A) Control and ENDOG knockdown cells were infected by VSV (MOI=0.2). At 24h post infection, cells were fixed for IF staining. Representative confocal IF images of γH2AX IF staining in control- and *ENDOG*-knockdown A549 cells were shown (scale bar: 10 µm). The bar graph shows quantification of the percentage of γH2AX positive cells by ImageJ from three independent experiments (mean ± SEM, ** P < 0.01). (2B) ENDOG knockdown cells were transfected with the plasmid of ENDOG deleted of mitochondrial targeting sequence (ΔMTS-ENDOG). These cells were infected with VSV (MOI=0.2). At 16h post infection, cells were fixed for γH2AX IF staining. Representative images and quantification of percentage of γH2AX positive cells are shown (n=3). (2C-2D) The release of ENDOG from mitochondria after VSV infection. Cells were infected by VSV (MOI=1) for 1h. At 8h post infection, cells were fixed for ENDOG and TOM20 IF staining. (2C) Representative confocal IF images of ENDOG and TOM20 staining in mock- and VSV-infected A549 cells are shown (scale bar: 10 µm). The bar graph shows the quantification of ENDOG intensity outside mitochondria by ImageJ from three independent experiments (mean ± SEM; *** P<0.005). (2D) Western blot analysis of VSV-N and ENDOG in mitochondrial fraction and cytosolic fraction with 0.3% of whole-cell lysates corresponding to the loading amount of mitochondria fraction. TOM20 and TUBB were loading controls for the fractionation. (2E-2G) ENDOG is a host fact that restricts VSV propagation. Control LacZ and ENDOG knockdown A549 cells were infected by VSV (MOI=1) for 1h. At 8h, cells were harvested for (2E) RT-qPCR and (2F) the medium for plaque assay. Fold changes in viral gene (*VSV-G*) levels in mock- or VSV-infected A549 cells and PFU/ml from three independent experiments are shown (mean ± SEM, * P < 0.05; NS: not significant). (2G) Cells at the indicated time were fixed for IF staining using dsRNA and COX4 antibody. Left shows the representative IF images. Right shows the quantified data of the dsRNA signal intensity by ImageJ from three independent experiments. (mean ± SEM, * P < 0.05; NS: not significant). (2H) A summary of two different effects of ENDOG released from mitochondria on host cells after virus infection.

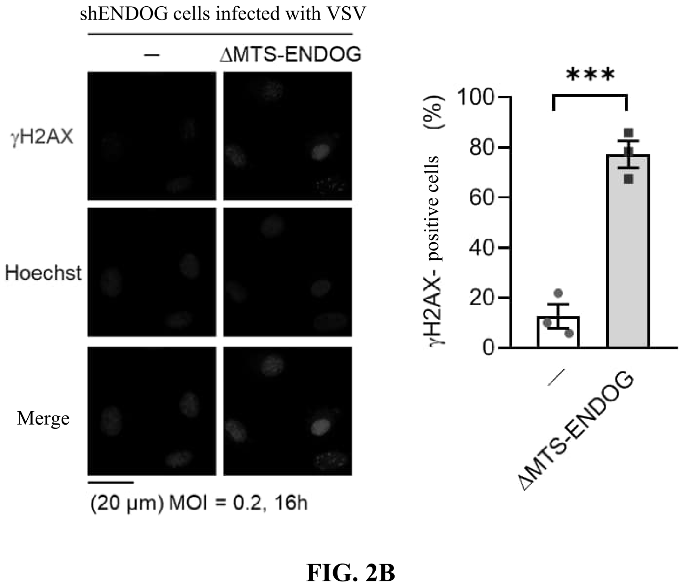

Several studies have shown that ENDOG, which normally reside in intermembrane space, is released to cytosol and translocated to nucleus under mitochondrial stress. We have previously shown that genotoxicity causes ENDOG leakage and translocation into nuclei to generate secondary nuclear DNA damages, thus intensifying DNA lesions to increase cell death. Here we observed that VSV infection also caused nuclear DNA damages as indicated by γH2AX IF staining, which was not seen in *ENDOG* knockdown cells (FIG. 2A). Expression of ENDOG outside mitochondria by deleting its mitochondrial targeting sequence (MTS) in ENDOG knockdown cells restored γH2AX signal in nuclei (FIG. 2B). Together with the observation that VSV infection causes the formation of mitochondrial spheres from the network, these data led us propose that ENDOG might leak into the cytosol in host cells after VSV infection to cause nuclear DNA damage.

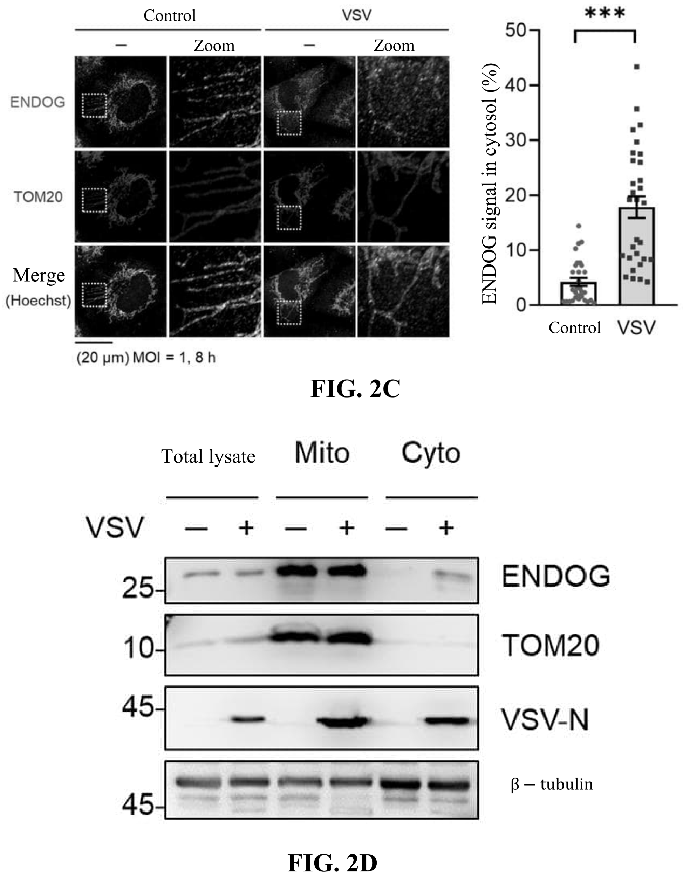

We then examined ENDOG subcellular localization in response to VSV infection. The results of IF staining revealed the detection of ENDOG outside of mitochondria in VSV infected cells (FIG. 2C). The biochemical fractionation confirmed a small fraction of ENDOG present in the cytosol (FIG. 2D).

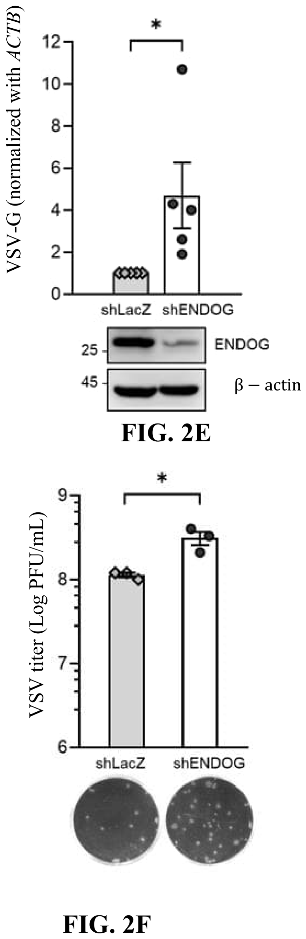

Since ENDOG is known to digest RNAs and DNAs and aforementioned viral RNA synthesis at mitochondrial spheres, we then tested the role of ENDOG in viral RNA expression. To this end, we measured viral RNA level in ENDOG knockdown cells after infection and found that the level of VSV-G RNA transcript was increased by ENDOG knockdown (FIG. 2E). Consistently, the plaque assay showed that ENDOG knockdown increased virus production (FIG. 2F).

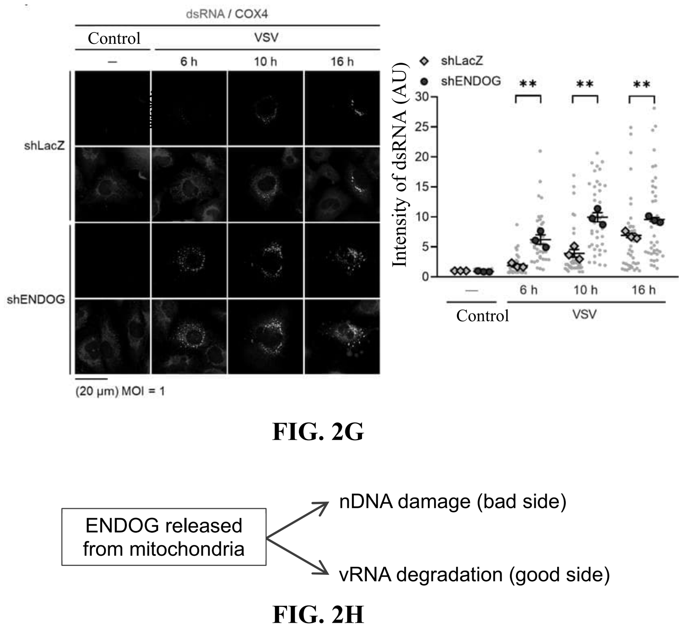

We further performed dsRNA IF staining that indicates viral transcription and replication. After infection from 6-16h, the intensity of dsRNA in spherical puncta was steadily increased and colocalized with mitochondria (FIG. 2G). The dsRNA intensity was higher in ENDOG knockdown cells, suggesting that ENDOG is a host factor that can reduce viral RNA, thereby suppressing viral propagation.

Gathering these data, we proposed that ENDOG released from mitochondria exerts two different effects on the host cells, one causes nuclear DNA damage, a bad side, and another degrades viral RNA, a good side for the host cells (FIG. 2H).

### Example 3

### Engineering an ENDOG expressed on mitochondrial surface, MOM-ENDOG

FIGs. 3A-3C show engineering an ENDOG, mitochondrial outer membrane (MOM)-ENDOG, expressed on mitochondrial surface. (3A) The design of ENDOG expressed on mitochondrial surface. The expression of vector of MOM-ENDOG was constructed by replacing the mitochondrial targeting sequence (MTS) of ENDOG-V5 by the N-terminal sequence of TOM20 (TN: SEQ ID NO:1), thus allowing ENDOG expressed on mitochondrial outer membrane (MOM). (3B) Representative super-resolution IF images of A549 cells transfected with TN-ENDOG^{ΔMTS}-V5 (MOM-ENDOG) by V5/TOM20 and V5/COX4 are shown (scale bar: 20 µm). (3C). Mitochondria fractions isolated from MOM-ENDOG-overexpressing A549 cells were incubated with proteinase K as indicated for 30 min in the presence and absence of 0.1% TritonX-100 for Western blot analysis using antibodies of the mitochondrial outer-membrane protein TOM20, matrix proteins and pyruvate dehydrogenases (PDHα/β).

Considering the expression of ENDOG deleted of mitochondrial targeting sequence (MTS) is harmful to nuclear DNA, we then engineered ENDOG anchoring on mitochondrial surface to increase its local access to VSV RNA transcripts while avoiding its translocation to nucleus. To this end, we replaced mitochondrial targeting signal (MTS) sequence of ENDOG by the sequence coding N-terminal 30 amino-acid of TOM20 (TN), which is a mitochondrial outer membrane (MOM) protein. The N terminal 30-amino-acid of TOM20 contains a transmembrane sequence that mediates its MOM binding. The engineered product is termed MOM-ENDOG. We also inserted a V5 tag at the C-terminus of MOM-ENDOG for the detection assay (FIG. 3A). Presumably, the construction allows MOM-ENDOG expressed on mitochondrial outer membranes facing the cytosol.

To assure that MOM-ENDOG is localized on mitochondrial outer membrane, we performed immunofluorescence (IF) co-staining of V5, indicating MOM-ENDOG, with TOM20 and COX-IV, indicating mitochondrial outer-membrane and inner-membrane marker, respectively. The IF image acquired by super-resolution microcopy revealed MOM-ENDOG co-localized with TOM20, but not COX-IV (FIG. 3B), indicating MOM-ENDOG localized on mitochondrial surface.

The biochemical analysis indicates that MOM-ENDOG-V5 and TOM20 in the mitochondrial fraction isolated from A549 cells were sensitive to proteinase K treatment, while matrix proteins, pyruvate dehydrogenase (PDH) α and β were insensitive until the incubation in the presence of 0.2% Triton X-100 (FIG. 3C), further confirming that MOM-ENDOG is expression on mitochondrial surface not inside mitochondria.

### Example 4

### MOM-ENDOG inhibits viral N gene expression via the nuclease function

We then examined whether MOM-ENDOG retains the nuclease activity in degradation of vRNA. To this end, VSV N-gene was cloned to an SP64 plasmid, allowing the in vitro transcription of N-RNA from the SP6 promoter. The increased amounts of wild-type (WT) and catalytic-dead (H141A) MOM-ENDOG immunoprecipitates were incubated with N-RNA. This in vitro degradation assay showed that WT but not H141A mutant of MOM-ENDOG is capable of degrading viral RNA (FIG. 4A).

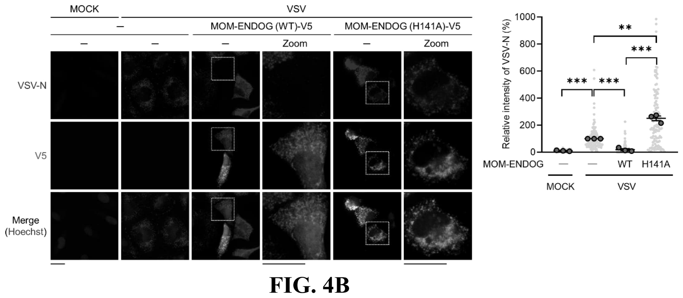

We further examined whether the expression of MOM-ENDOG is able to block viral N protein expression after virus infection in a catalytic-dependent manner. Cells expressing WT and H141 mutant of MOM-ENDOG-V5 were infected by VSV. The expression of VSV N protein expression from virus infection was examined by IF staining. We found that N protein puncta staining was all over the cytoplasm in infected cells. In WT but not H141A mutant MOM-ENDOG-V5 positive cells, viral N protein signal was completely abolished. Thus, MOM-ENDOG might eradicate viral RNA on mitochondrial surface, thus preventing viral protein synthesis (FIG. 4B).

### Example 5

### Delivery of modified mRNA of MOM-ENDOG-polyA³⁰ represses VSV production and VSV-induced nDNA damage

FIGs. 5A-5E show that delivery of the modified mRNA of MOM-ENDOG containing polyA³⁰ tail suppresses VSV propagation without causing nuclear DNA damage. (5A) The generation of MOM-ENDOG modified mRNA. A schematic illustration for generation of MOM-ENDOG modified mRNA containing 5' capping, polyA³⁰ and pseudoUridine (ψ). (5B) A549 cells transfected with 400 ng of modified mRNAs of MOM-GFP or MOM-ENDOG were harvested at indicated time for Western blot. (5C-5E) Cells were infected with VSV (MOI=1) for 1 h and then transfected with modified mRNA of MOM-GFP, WT- and H141A- MOM-ENDOG. (5C) The culture media were collected at 8 h post infection for plaque assay. Plaque forming units (PFUs) of media were determined. Data from three independent experiments are presented (mean ± SEM, * P < 0.05). The expressed levels of MOM-GFP, WT- and H141A-ENDOG were shown by Western blots. (5D) Cells were fixed for dsRNA IF staining. Representative confocal IF images of dsRNA and V5 IF staining at 20 h after mock- or VSV-infection are shown (scale bar: 10 µm). The bar graph shows relative dsRNA signal intensity in transfected cells quantified by ImageJ from three independent experiments (mean ± SEM, ** P < 0.01). (5E) LacZ control and ENDOG-knockdown A549 cells were transfected wit modified mRNAs of MOM-GFP or MOM-ENDOG and infected by virus. At 24h post infection, cells were fixed for γH2AX IF staining. Representative confocal IF images are shown (scale bar: 10 µm). The bar graph shows quantification of the percentage of γH2AX positive cells by ImageJ from three independent experiments (mean ± SEM, ** P < 0.01).

To test the potential of MOM-ENDOG in a form of modified mRNA as a therapeutic agent against virus infection, We then inserted MOM-ENDOG to a pSP64-PolyA³⁰ vector to synthesize modified mRNA by incorporating 3'-O-Me-m7G(5')ppp(5')G RNA cap structure analog and pseudouridine for the cellular delivery of modified mRNA containing a length of 30 adenylate at the 3'end for protein expression in the cells (FIG. 5A). In this study, we also generated MOM-GFP modified mRNA as a control. The levels of expressed proteins from delivered MOM-GFP- and MOM-ENDOG-polyA³⁰ modified mRNA were well detected at 8 h but declined at 16 h post transfection (FIG. 5B).

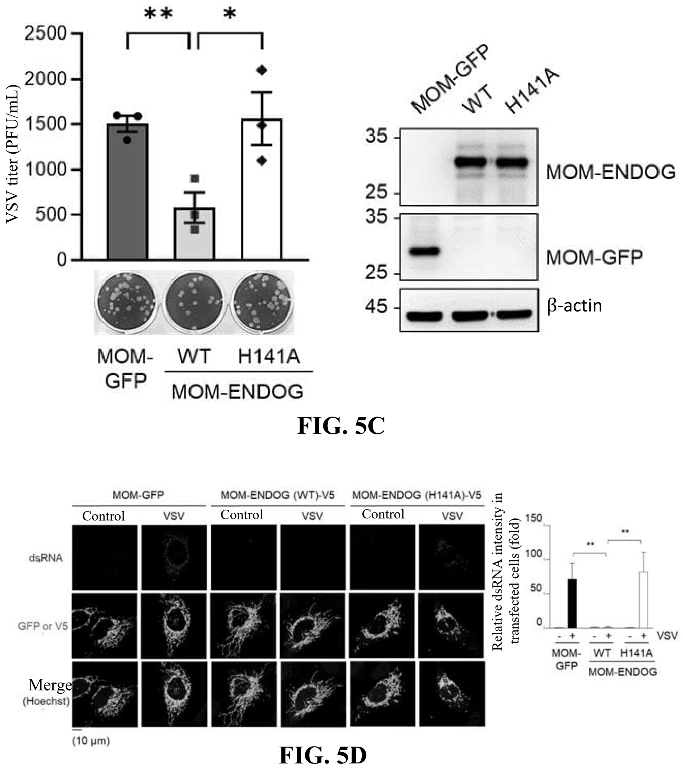

Cells after virus infection for 1h were transfected with modified mRNA of MOM-GFP-, WT-, and H141A-MOM-ENDOG-polyA³⁰. The plaque assay indicated the delivery of WT-, but not H141A-MOM-ENDOG-polyA³⁰ was able to suppress virus production (FIG. 5C).

VSV-mediated dsRNA signal in the cytosol that indicates viral RNA transcription and replication was clearly detected in the cells expressing MOM-GFP or MOM-ENDOG(H141A)-V5 after virus infection. In contrast, these virus-induced dsRNA signals were not detectable in cells expressing WT MOM-ENDOG-V5 (FIG. 5D).

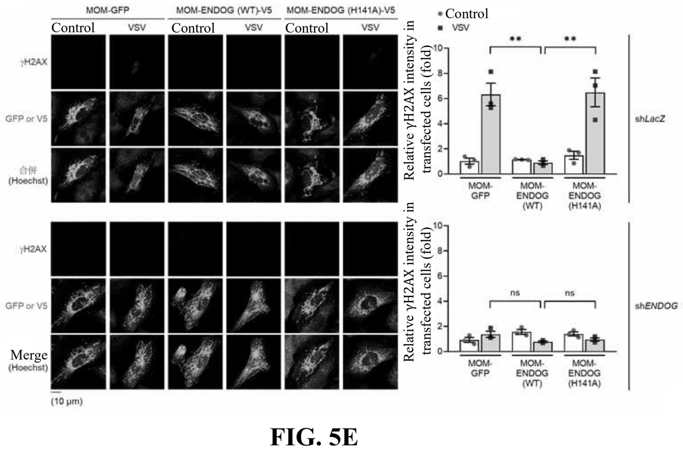

We further compared the effect of the delivery of MOM-GFP, MOM-ENDOG, and MOM-ENDOG (H141A) modified mRNA on VSV-induced nuclear DNA damages in control and *ENDOG* knockdown cells. In VSV infection control cells expressing MOM-GFP or MOM-ENDOG(H141A), γH2AX DNA damage signals were detected in nuclei but not in *ENDOG* knockdown cells, suggesting the contribution of endogenous ENDOG in DNA damage. We did not find γH2AX DNA damage signal in nuclei of the cells expressing MOM-ENDOG-V5 regardless of control cells or *ENDOG* knockdown cells (FIG. 5E). Thus, the delivery of MOM-ENDOG-poly(A³⁰) modified mRNA not only prevents viral RNA replication but also avoids nuclear DNA damage.

### Example 6

### Prolonged duration of MOM-ENDOG expression markedly reduces virus production

FIGs. 6A-6E show that prolonged duration of MOM-ENDOG markedly reduces VSV virus production. (6A) The design of capping modified mRNA of MOM-ENDOG containing polyA¹³⁰ tail. (6B) A549 cells transfected with 400 ng of capping modified mRNA of WT and H141A-MOM-ENDOG containing polyA¹³⁰tail were harvested at the indicated times for Western blot analysis. (6C-6D). Cells transfected with modified mRNA overnight were infected by VSV (MOI=0.2) for 1h. Cells and medium were harvested at 24h post-infection for (6C) rt-qPCR of viral genes (VSV-G and VSV-M) and (6D) plaque assay. E. The levels of mt-tRNA, mt-ND4, mtND6, 18S RNA by rt-qPCR in cells transfected with MOM-ENDOG-polyA¹³⁰ for 24h. Data are presented in relative amount normalized by β-actin from three independent experiments (NS, not significant).

Aforementioned modified mRNA containing A³⁰ tail, the duration of expressed protein lasted 16h. We then constructed the modified mRNA containing poly(A¹³⁰) tail (FIG. 6A). The duration of MOM-ENDOG (WT) and (H141A) sustained to 48h (FIG. 6B).

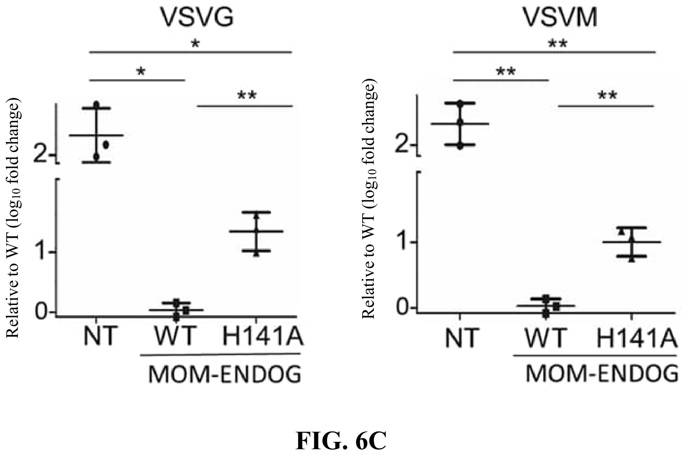

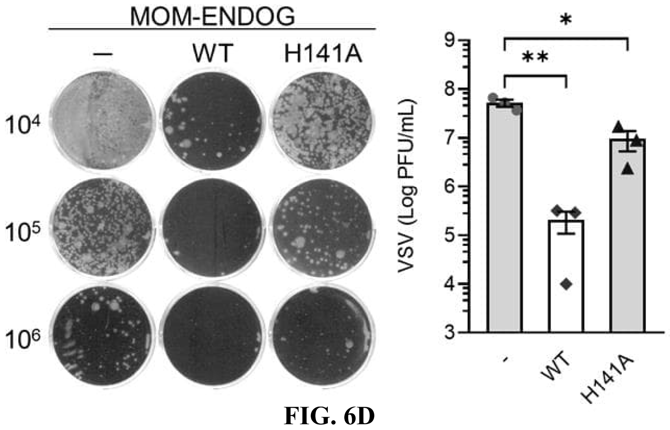

We then examined the anti-viral effect of these modified mRNA with longer duration of protein expression. After VSV infection, RT-qPCR and plaque assays indicated that the delivery of WT MOM-ENDOG modified mRNA containing polyA¹³⁰ tail caused a 100-fold reduction in the levels of VSV-G and -M transcripts and virus production. Clearly, the delivery of modified mRNA of MOM-ENDOG with longer polyA¹³⁰ tail prolongs the duration markedly impacts virus production. Since the delivery of H141A MOM-ENDOG modified RNA did not cause such a striking difference, the anti-viral effect of MOM-ENDOG expressed from modified mRNA is through its function in nucleic acid degradation (FIGs. 6C and 6D).

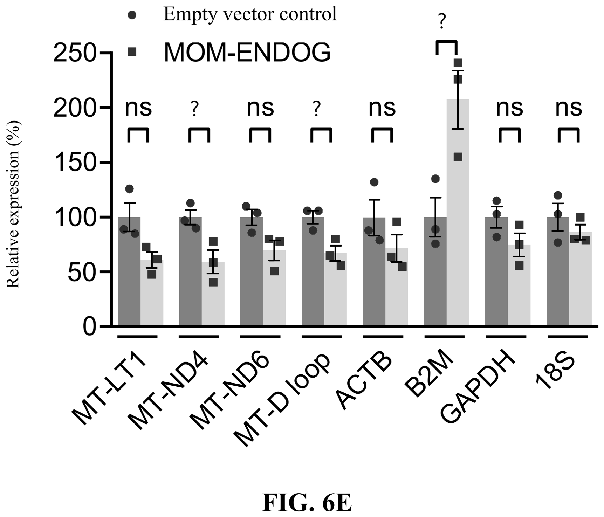

Of note, the delivery of MOM-ENDOG-poly(A¹³⁰) modified mRNA for 48h did not affect the RNA levels of housekeeping genes such as *ACTB, 18S, MT-tRNA, MT-ND4,* and *MT-ND6* (FIG. 6E). Altogether, we conclude that MOM-ENDOG on mitochondrial surface can act as a viral RNA degrader, thus suppressing VSV propagation.

### Example 7

### MOM-ENDOG exerts a broad-spectrum of anti-viral effect

FIGs. 7A-7D show that delivery of the modified mRNA of MOM-ENDOG containing polyA¹³⁰ tail markedly suppresses ZiKa. Dengue and Influenza virus propagation. (7A) A549 cells transfected with 400 ng of capping modified mRNA of WT and H141A-MOM-ENDOG containing polyA¹³⁰ tail. Cells were then infected by Zika virus (ZIKV, MOI=0.1) for 1h. Cells and medium were harvested at 24h post-infection for plaque assay. B) A549 cells transfected with the modified mRNA as described above, and infected by Dengue virus 2 (DENV2, MOI=0.1) for 1h. Cells and medium were harvested at 24h post-infection fo plaque assay. Data are from three experiments (mean ± SEM, ***P< 0.005). We also delivered WT and H141A MOM-ENDOG-polyA¹³⁰ to A549 cells and infected these cells with the Influenza virus strain H1N1. Plaque assay showed that H1N1 virus production in these cells decreased by approximately 5-fold after WT delivery, while no reduction was observed after H141A MOM-ENDOG-polyA¹³⁰ delivery. (7D) The proposed mechanism for MOM-ENDOG-mediated anti-virus activity. After cellular entry, the disassembly of RNA virus releases vRNA and induces mitochondrial spheres as vRNA production factories. MOM-ENDOG expressed on mitochondrial surface by the delivered modified mRNA degrades accessible vRNAs, thus terminating the propagation of vRNA replication.

To know whether the antiviral effect of ENDOG can be extended to other RNA viruses. We delivered modified mRNA of MOM-ENDOG (WT) and (H141A) containing A¹³⁰ tail. Cells were infected with Zika virus for 1h. The focus forming assays indicated that the delivery of WT but not H141A mutant of MOM-ENDOG modified mRNA caused a 66-fold reduction in virus titer (FIG. 7A).

As for Dengue virus, the focus forming assays showed that the delivery of WT but not H141A mutant of MOM-ENDOG modified mRNA caused a 90-fold reduction in virus titer (FIG. 7B).

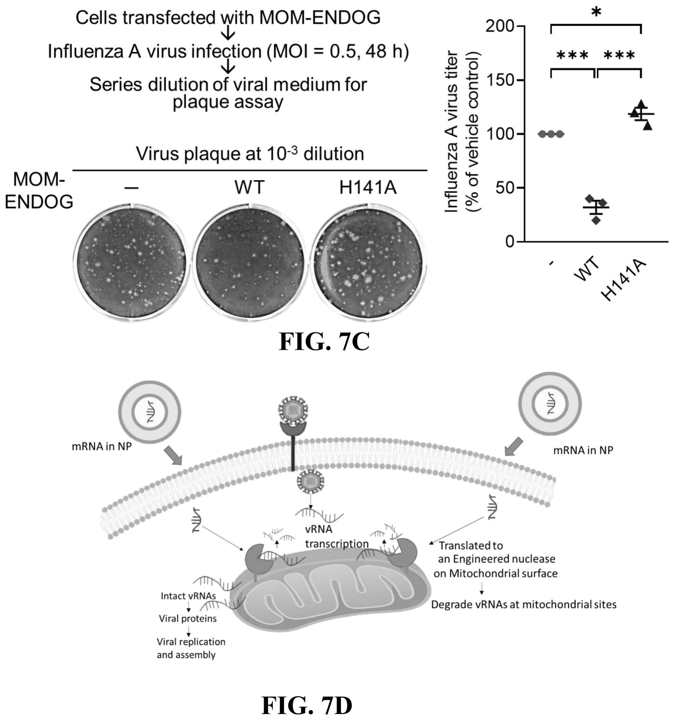

For Influenza virus (H1N1), the plaque assay showed that the delivery of WT but not H141A mutant of MOM-ENDOG modified mRNA caused about 5-fold reduction in virus titer (FIG. 7C).

Thus, MOM-ENDOG exerts a broad-spectrum of anti-viral effect. We propose that viral RNA at the sites of mitochondrial surface is susceptible to MOM-ENDOG, which degrades vRNAs to terminate the biological processes for viral propagation (FIG. 7D).

In summary, the present invention achieves the effect of treating RNA virus infection through the results illustrated in the following examples. In particular, by linking to polypeptide sequences, proteins that can break down nucleic acids or inhibit nucleic acid synthesis can be engineered to anchor on organelle surfaces. Therefore, engineered enzymes directly and negatively impact the nucleic acid synthesis of invaded viruses at the neighborhood site of the organelle in host cells.

This method differs from traditional antiviral small molecule drugs in that it can suppress the propagation of different types of viruses, and is not affected by viral gene mutations or evolution. This organelle-associated vRNA degrader is a drug that combats a variety of viruses and can overcome drug-resistant RNA viruses.

Although the present invention has been described with reference to the preferred embodiments, it will be apparent to those skilled in the art that a variety of modifications and changes in form and detail may be made without departing from the scope of the present invention defined by the appended claims.

## Claims

1. A blocking agent that suppresses virus propagation for treating and/or mitigating virus infection, comprising a polypeptide and an enzyme conjugated to the polypeptide, wherein the blocking agent is expressed on a surface of an organelle.

2. The blocking agent according to claim 1, wherein the polypeptide is conjugated to the enzyme, anchoring the enzyme to the surface of the organelle, thereby reducing nucleic acid synthesis of viruses at the neighborhood site of the organelle in host cells.

3. The blocking agent according to claim 2, wherein the enzyme is anchored to an outer membrane of the organelle.

4. The blocking agent according to claim 1, which inhibits viral production without damaging nuclear DNA (nDNA).

5. The blocking agent according to claim 1, wherein the virus is an RNA virus or a DNA virus.

6. The blocking agent according to claim 5, wherein the RNA virus is selected from the group consisting of: dengue virus, Zika virus, vesicular stomatitis virus (VSV), Influenza virus, respiratory syncytial virus (RSV), and a combination thereof.

7. The blocking agent according to claim 3, wherein the organelle is a mitochondrion, endoplasmic reticulum, or Golgi apparatus.

8. The blocking agent according to claim 1, wherein the polypeptide is an N-terminal sequence of translocase of outer mitochondrial membrane 20 (TOM20).

9. The blocking agent according to claim 8, wherein the polypeptide comprises an amino acid sequence of SEQ ID NO:1.

10. The blocking agent according to claim 1, wherein the enzyme is endonuclease G (ENDOG).

11. The blocking agent according to claim 7, wherein the surface of the organelle is an outer membrane of mitochondria.

12. The blocking agent according to any one of claims 1-11 for use in the treatment and/or mitigation of virus infection.

13. The blocking agent for use according to claim 12, wherein the blocking agent is in a dosage form for oral administration.

14. The blocking agent for use according to claim 12, wherein the blocking agent is in a dosage form for parenteral administration.

15. A synthetic nucleic acid, encoding the blocking agent according to claim 1, wherein the synthetic nucleic acid is in the form of a DNA or an RNA.
